# EUROPEAN PATENT APPLICATION

(11) **EP 4 385 458 A1**
(43) Date of publication of application: **19.06.2024**
(21) Application number: 22213584.0
(22) Date of filing: 14.12.2022
(51) Int. Cl.: A61C 19/06

(54) **ORAL FILM WITH MULTIPLE REGIONS FOR THERAPY OF PERIODONTAL AND DENTAL DISEASES OR DISORDERS**

(71) Applicant: vVardis AG, 6340 Baar (CH)
(72) Inventor: HUG, Michael, 5210 Windisch (CH); CAZARES DELGADILLO, Jennyfer, 5210 Windisch (CH); VENTURATO, Andrea, 5210 Windisch (CH); HOFFMANN, Waldemar, 79807 Lottstetten (DE); ABIVARDI BRÖNNER, Haleh, 6340 Baar (CH); ABIVARDI SIGNER, Golnar, 6340 Baar (CH); LYSEK, Dominikus Amadeus, 5210 Windisch (CH)
(74) Representative: Moré, Solveig Helga

(57) **Abstract**

The present invention relates to the field of dental care products used for dental diseases, periodontal diseases and or any other oral disorders, or for cosmetic therapies. It provides an oral film, in particular a pharmaceutical oral film, that can be used for simultaneous therapy of a gum and teeth of a subject, e.g., for therapy and/or prevention of periodontal and dental diseases or disorders, or for cosmetic therapies such as for tooth whitening. The invention also provides a method, e.g., a cosmetic method using said film and a method for preparing said oral film.

## Description

The present invention relates to the field of dental care products used for therapy and/or prevention of periodontal and dental diseases or disorders, or for cosmetic therapies. It provides an oral film, in particular a pharmaceutical oral film, that can be used for simultaneous therapy of a gum and teeth of a subject, e.g., for therapy and/or prevention of dental diseases, periodontal diseases and or any other oral disorders, or for cosmetic therapies such as for tooth whitening. The invention also provides a method, e.g., a cosmetic method using said film and a method for preparing said oral film.

Oral films for preventive, restorative and regenerative therapies of teeth gained a wide range of applications in dentistry due to their adaptable mechanical and biological properties, low production cost and ease of processing. They enable local action, rapid release of agents or act as buccal adhesive systems retained in the oral cavity for controlled drug release. Oral polymeric films are often used in personal care products and over-the-counter medications.

One common application of oral films, also designated "strips" are for use in tooth whitening (e.g., US 6,893,629 B2, US 10,258,546 B2, WO 2021/224618, EP 3 315 118 A1, US 10, 849,729 B2, US 9,782,338 B2, US 9,149, 417 B2, EP 3 302 405 A1, US 6,949,240 B2, EP 1696 866 A1, EP 2 934 446 A1, US 9,668,970 B2).

Both extrinsic and intrinsic reasons contribute to discoloration of teeth. For example, caused by lifestyle habits or food e.g., consummation of coffee, tea, wine, carrots, oranges or tobacco can leave stains on the tooth surface. The use of certain dental care products like mouth rinses containing chlorhexidine and cetylpyridinium chloride or dental materials like amalgam, especially silver sulphide-containing materials can also lead to extrinsic induced tooth discoloration. Further, certain medications, therapies, or hereditary diseases may cause intrinsic discoloration by preventing normal development of tooth enamel and dentin. Some of these medications leading to tooth discolorations are antibiotics like tetracycline and doxycycline or antihistamines, antipsychotic drugs and antihypertensive medication, or excessive fluoride uptake. Even some infections in pregnant mothers can affect enamel development of their baby. Certain medical treatments, such as head and neck radiation and chemotherapy can also lead to tooth discoloration. Also tooth trauma may lead to discoloration. With time, the outer layer of enamel on the teeth gets worn away and exposes the yellow dentin with age.

The tooth dentin also grows with aging, which decreases the size of the pulp. Therefore, the translucency of the tooth is reduced, making it look darker. Tooth discoloration can be an important aesthetic problem for dental patients. For example, in the UK, around 20% of people are dissatisfied with their teeth colour. In the USA, 34 % seem to be dissatisfied. According to the American Academy of Cosmetic Dentistry, 99% of the population consider their smile as their most important social feature.

For example, US2004062724A1 discloses a bi-layered or multi-layered strip comprising an enamel adherent, water soluble, polymeric layer containing a tooth whitening agent or other active compounds and a coated erodible backing layer that controls the desired residence time.

US20070178055A1 discloses a dissolvable tooth whitening strip comprising a whitening agent and a water-soluble polymer system. The whitening strip includes additional ingredients, some of which are active agents, to provided additional benefits such as gum health maintenance or treatment. In addition, several agents are comprised in the whitening strip such as antimicrobial agents, mineralization compounds, desensitization compounds, anti-calculus agents, flavouring agents, anti-inflammatory agents, antioxidants and vitamins.

Whitening products often contain peroxide-based agents, which are very reactive and can both harm the enamel and irritate the gum.

In light of this, the inventors addressed the problem of providing an advantageous oral film that is suitable for simultaneous administration of active agents to the teeth and gum. It may be used for local and controlled delivery of cosmetic and/or pharmaceutical active agents, e.g., for tooth whitening, and/or treating, preventing or reducing incidence of dental caries, tooth decay, mucositis, gingivitis, periodontitis, peri-implantitis or tooth discoloration. Advantageously, it provides more flexibility in the choice of active agents than systems known in the art. The problem is solved by the invention, in particular, by the subject-matter of the claims.

The invention provides a film comprising a first surface comprising at least one first region comprising a first active agent and at least one second region comprising a second active agent different from the first active agent,
wherein the at least one first region is capable of contacting at least one tooth of a subject and the at least one second region is capable of simultaneously contacting a gum of the subject.

The film of the invention is a film having a length, a width and a thickness. The thickness has the smallest extension. The film can be, e.g., 1-3000 µm thick, e.g., 20-2000 mm, 40-1000 µm, 50-800 µm, e.g. 100-500 µm or 100-200 µm.

The first surface typically extends over the length and width of the film. It is the surface in which the first region and second region are arranged. Typically, the first and the second region are adjacent to each other, i.e., the first region is adjacent to the second region in the first surface.

The at least one first region is capable of contacting at least one tooth of a subject and the at least one second region is capable of simultaneously contacting a gum of the subject. Thus, the first region can comprise at least one active agent suitable for acting on a tooth, e.g., a tooth whitening agent such as a peroxide or an agent capable of releasing peroxides, an anticaries agent and/or a desensitizing agent such as a self-assembling peptide (e.g., P11-4), and the second region can comprise at least one active agent suitable for acting on a gum, e.g., an anti-inflammatory agent, an anti-irritant agent and/or a senolytic agent. For example, the second agent can comprise an agent capable of neutralizing free radicals. In a classical tooth whitening strip comprising peroxides or agents capable of releasing peroxides, such an agent would reduce the desired bleaching activity on the tooth. In the oral film of the invention this is not the case, because both agents are applied in a targeted manner. In addition to direct incompatibilities of active agents, there are several agents beneficial for therapeutic treatment of the gums that may lead to tooth discoloration if they are applied to teeth, which is avoided or minimized if they are targeted to the gums with the oral film of the present invention. The present invention thus allows for more targeted treatment of teeth and gums and for more flexibility in the selection of active agents.

The first region of the film of the invention may have a size suitable for contacting one tooth, i.e., the length and width may correspond to the length and width of a tooth. For example, a first region may have an area of 0.5-2 cm * 0.5 -2 cm. Typically, it is suitable for simultaneously contacting a plurality of teeth. Preferably, it is suitable for contacting at least the labial or vestibular side of the incisors, and, optionally, the canines. It may also be suitable for contacting the labial/vestibular or buccal side of the upper or lower teeth, or for contacting the palatinal side of the incisors, optionally, the incisors and canines or even the incisors, canines and one or more, e.g., all premolars or molars of the upper or lower teeth. Thus, the first region of the film of the invention may have a length of one tooth, e.g., about 0.5-2 cm (e.g., 1-1.5 cm) and a width of 3-25 cm, e.g., 4-22 cm, 5-20 cm or 7-10 cm. It may also be designed for the subject cutting a suitable width, and optionally, a suitable length from a bigger film.

In one embodiment, the film has a first region sized to contact not only the labial/vestibular or buccal side of a tooth or teeth, but it can be fitted around the tooth or around neighbouring teeth, so that the first region contacts both the labial/vestibular or buccal side and (and for the premolars or molars, the occlusal side. In this case, there can be one second region suitable for contacting the gum on the labial/vestibular/buccal side, and, optionally, there can be a further second region contacting the gum on the palatine side, i.e., on the opposite side of the first region. For this embodiment, the length of the first region may be about 1-4 cm (e.g., 2-3 cm) and the width may be, e.g., 3-25 cm, e.g., 4-22 cm, 5-20 cm or 7-10 cm.

In another embodiment, the film may be designed to simultaneously contact the labial/vestibular, and optionally, buccal side of both upper and lower teeth. In this case, also, two second regions on opposite sides of the first region may be present.

Typically, permanent teeth will be treated with the oral firm of the invention, but for treatment of deciduous teeth, the sizes may be even smaller.

There may generally be a plurality of first and second regions, which can also be separated by regions without active agents, but each first region is adjacent to at least one second region.

The second region typically extends along the width of the first region. The length of the second region may be, e.g., 0.1 to 2 cm, e.g., 0.2-1 cm or 0.3 to 0.5 cm.

The film may be individualized, or it may be possible to individualize it for a specific subject and said subject's shape and size of teeth. The exact measures of the film or the first and second regions thereof may be selected for a particular subject, typically, based on the size of the incisors. If treatment of other teeth is of higher importance, said teeth may be determine the size of the firm employed.

The form of the first region is typically rectangular for ease of fabrication and standardization. In that case, because of the curve of the gingival border, there may be, upon application to the teeth and gum, some contact between the first region and the gum and/or between the second region and the tooth or teeth, however, the treatment is overall more targeted than treatment with a film only comprising one region. The film can alternatively be more specifically fitted to the form of the teeth and, in particular, the curve or curves of the gingival border. This allows still more targeted treatment of teeth and gums.

The oral film may be flat or essentially flat. It may also have at least one recess, at least one elevation, perforations or any other kind of microstructure. For example, its topography may be configured to physically bind active agents within one region of the film. The height of the regions due to such elevations or microstructure is included in the thickness of the film.

In the context of the present invention, gum is defined as the periodontium, i.e., the connective tissues consisting of cementum, periodontal ligament, alveolar bone and gingival tissue. The periodontium surrounds and supports the teeth. Gum it is used synonymously with gingiva in this application.

Teeth can be any teeth, and in principle, any side of these teeth. In particular for whitening applications, typically, labial sides of the incisors and, optionally, vestibular sides of canines are contacted with the oral film of the invention, but this can also extend to the buccal side of molars. Other or additional sides of the teeth can also be contacted, e.g., in the context of caries treatment or prevention or treatment of periodontitis or aging of gums and teeth. In the context of the present invention, if not specifically mentioned otherwise, application to a tooth includes application to a plurality of teeth.

The subject will typically be a human subject, e.g., an adult or a teenager. The subject may also be an aging person, e.g., a person 50 years old or older. A child may also be treated. The subject may also be an animal, e.g., a dog, a horse or a camel.

The oral film of the invention preferably is dissolvable after administration of said film in the oral cavity, in particular, administration to teeth and to the gum. For example, the film may be substantially dissolved 1-240 min, e.g., after 2-180 min, 3-120 min, 4-90 min, 5-60 min, 8-45 min, 10-30 min, or 15-20 min after administration. Preferably, this is the case after 15 min or more to allow for sufficient contact times with the active agents. Substantially dissolved means that the continuity of the film is lost, and more than 50%, more than 60%, more than 70%, more than 75%, more than 80%, preferably, more than 90% or, optionally, more than 95% of the film's area are dissolved. Of course, the film, can also be totally dissolved in this time. The residence time (i.e., time until the film is substantially dissolved) of the film is thus determined by the solvability of the film. Optimal residence time depends on the desired, e.g., therapeutic or cosmetic application and/or on the active agents contacted to the teeth and gum.

Optionally, the film comprises a backing layer that forms the second surface of the film opposite to the first surface. Such a backing layer may, e.g., make residence times longer, e.g., if the backing layer dissolves more slowly than the material of the first and second region. If the backing layer hinders diffusion of one or more active agents, it may also minimize exposure of the lips or tongue to the active agents in the sheet, which is advantageous not only in case one or more active materials are irritants (such as peroxides or agents capable of providing a peroxide), but may also help to increase the concentration of active agents at the teeth and gum, respectively.

There are different options for configuration of an oral film of the invention.

In one embodiment, the first region is formed by a first sheet and the second region is formed by a second sheet, wherein the surfaces of said first and second sheets together form the first surface. Accordingly, the film does not have to be a continuous film, but two sheets adjacent two each other may form the two regions. This has the advantage that each sheet (forming the first or second region) comprising at least one different active agent may be prepared separately. Each sheet may be prepared, independent from another, by impregnating a ready-made sheet with at least one active agent, e.g., by soaking it in a solution of the active agent or adding active agent one the sheet. Alternatively, it can be prepared, e.g., polymerized, extruded or dried after mixing of a matrix material of the sheet with the at least one active agent.

When regions are formed by separate sheets, the first region may have a different residence time than the second region, e.g., the first sheet may have a different matrix material and/or a different solubility and/or a different thickness than the second sheet. This may be helpful if, e.g., treatment of the gums is desired for a longer time than treatment of the teeth, or vice versa.

Optionally, said first sheet or said second sheet may be overlapping 10-100% of the respective other region. This configuration may simplify preparation of the film of the invention. For example, in case there is no backing layer, it allows for a firm fixation of the two regions in one film. The structure is particularly firm if the overlap is 100%.

The sheets may, e.g., overlap by 3-100%, such as 5-50% and 10-15%.

In one embodiment, the first region overlaps the second region, e.g., by at least 3 %, e.g., by at least 50% or 100%. Thus, in this case, in the area of the second region, the first sheet forms a second layer opposite the first surface of the second region. This has the effect that the active agent(s) comprised in the first region may also be provided to the gums through the second region contacting the teeth. Optimally, in this case, the first region does not comprise an irritant agent. This may be advantageous if, instead it comprises an agent that may be beneficial for both gums and teeth, e.g., a self-assembling peptide such as P11-4.

In an alternative embodiment, the second region overlaps the first region, e.g., by at least 3 %, e.g., by at least 50% or 100%. Thus, in this case, in the area of the first region, the second sheet forms a second layer opposite the first surface of the first region. This has the effect that the active agent(s) comprised in the second region may also be provided to the teeth through the first region contacting the teeth. Optimally, in this case, the second region does not comprise agents problematic for the teeth, such as a colouring agent. This may be advantageous, if, instead an agent that may be beneficial for both gums and teeth is comprised, e.g., a self-assembling peptide such as P11-4.

If the first and the second sheet do not overlap, the film of the invention preferably comprises a backing layer to improve stability.

If the first and the second sheet do not overlap, they may be abutting each other.

Thus, they are in contact with each other with at least one of their narrow sides

Alternatively, the first and the second sheets may be distanced by a spacer region. The spacer region may be a region not containing an active agent. It may still be capable of mediating diffusion of an active agent if the film is placed in the oral cavity and/or contacted with water. Alternatively, said spacer region may be configured to inhibit diffusion of at least one of the active agents from one region into the other region, e.g., due to hydrophobic properties, or by the absence of material. In the latter case, the first surface of the spacer region may be formed by the backing layer.

For example, the spacer region may be configured to be placed over the gingival border of the teeth, e.g., if it is desired that an active agent in the first region is not administered to the gums and/or an active agent in the second region is not administered to the teeth.

The spacer region may be an area of film between at least one first and second sheet. The spacer region may be formed by at least one sheet (spacer sheet) arranged between the first and second sheets, abutting the narrow sides of the respective sheets. The spacer region optionally may also surround one of the first or second regions, or both.

Alternatively, the spacer sheet may overlap the first and second region in the first surface, i.e., at the position of the spacer sheet, the first and second regions do not form the first surface

In one embodiment, the first region, the second region, and, if present, the spacer region are regions of the same sheet forming the film. Accordingly, in this case, these regions are all part of a continuous sheet. The regions are thus made of one common material. In this case, typically, the film is prepared by adding the at least two different active agents to their respective regions. To separate the active agents in this embodiment, it can be helpful to include a spacer region that inhibits diffusion from one region into the other, e.g., by first impregnating the sheet with a hydrophobic material that inhibits diffusion of hydrophilic active agents.

The invention for example provides a film, wherein the first sheet and the second sheet forming the first surface are arranged on a backing layer forming a second surface opposite to the first surface. The backing layer supports both first and second sheets. If a spacer region is present, then the spacer region may be a non-covered region of the backing layer between or around the first and second regions. The spacer region may also overlap the sheets of the first and second regions as described in detail above, or it may abut with the first region on one side and the second region on the opposite side.

Regardless of the question if there is one or more sheets forming the first surface, the material of the backing layer is preferably configured to be dissolvable by water or saliva. It optionally comprises cellulose. The backing layer may have a residence time, dependent on its a solvability, different than the residence time, dependent on the solvability, of the sheet(s) or the film forming the first and second regions.

Optionally, the backing layer comprises at least one third region including an active agent, wherein the third region forms at least part of the second surface. The third region is suitable for contacting the lips and/or the tongue of the subject if the first region contacts the teeth and the second contacts the gum. The third region may comprise one of the active agents comprised in the first or second region, preferably, the same agent(s) comprised in the second region, but it may alternatively or additionally comprise a third agent different than the first and second agent.

Optionally, the backing layer is configured to inhibit diffusion of at least one of the active agents towards the oral mucosa of the subject. To this end, e.g., the backing layer can be less water-permeable and/or less permeable to the active agent(s) than the sheet or sheets forming the first and second regions. Preferably, the residence time of the backing layer is longer than the residence time of the sheet(s) of the first and second regions.

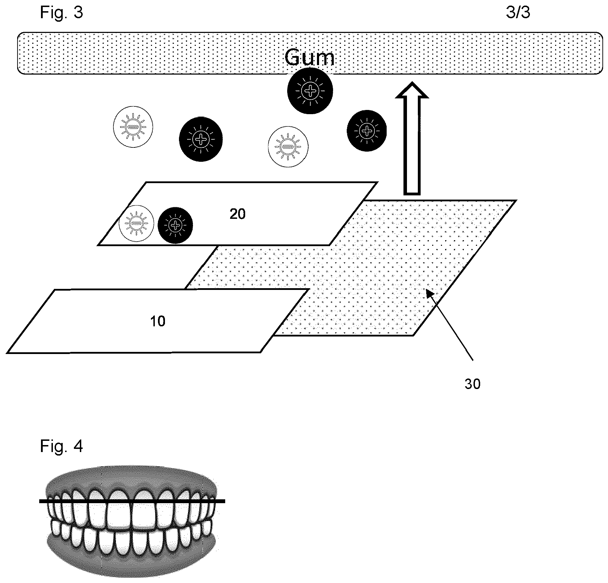

In the film of the invention, at least one selected from the group consisting of the backing layer, the spacer region, the first region, and the second region may be configured to release ions upon placement in an oral cavity. In one embodiment, the backing layer, which preferably reduces or inhibits diffusion of active agents from the second surface of the film towards the oral mucosa of the subject, is further capable of releasing ions, as e.g., shown in Fig. 3. The release of ions may be triggered, e.g., by the contact with water or saliva after placement in the oral cavity. The film can also be brought into contact with water before placement in the oral cavity to activate it. The release of ions may be used to locally alter the pH. For example, the pH can be made more acid by release of H⁺ ions. This may, e.g., be helpful in case it is desired to provide the assembled form of a self-assembling peptide to a tooth or teeth, wherein the self-assembling peptide assembled when the pH is acidic, e.g., for P11-4.

Alternatively, the pH can be made more basic by release of OH⁻ ions. This may, e.g., be helpful in case it is desired to provide the assembled form of a self-assembling peptide to a tooth or teeth, wherein the self-assembling peptide assembled when the pH is basic, e.g., for P11-8. Other ingredients' reaction on teeth and gums may also be fostered by release of ions from the backing layer.

Using the same principle, it may be an additional middle layer instead of the backing layer that is capable of releasing ions, wherein the backing layer is preferably reduces or inhibits diffusion of active agents from the second surface of the film towards the oral mucosa of the subject. The middle layer may be located between the backing layer and the sheet or sheets forming the first surface of the film.

Optionally, the film of the invention, e.g., the backing layer, comprises a battery element, being either a bio-battery or at least one other galvanic cell. The biobattery converts chemical energy from an energy carrier, typically, a sugar such as glucose or maltodextrin, preferably glucose, into electrical energy. It requires a suitable energy carrier, enzymes capable of converting it and water. It can be activated, e.g., by adding glucose, or by adding water (or saliva) to a dry biobattery, wherein e.g., the water (e.g., from the saliva) enables diffusion of the energy carrier towards the biobattery. The enzymes may be comprised in suitable bacteria.

Other galvanic cells include, e.g., a nanobattery. For example, lithium batteries may be used.

The battery, e.g., the bio battery, preferably is a primary cell comprising at least two electrodes that are separated by a membrane. Bio-batteries consist of an anode, cathode, separator and electrolyte with each component layered on top of another. At the anode, the sugar is oxidized, producing both electrons and protons. The electrons travel from the surface of the anode through an external circuit to get to the cathode. On the other hand, the protons are transferred via the electrolyte through the separator to the cathode side of the battery.

The cell of the battery may be planar. Multiple cells may be arranged adjacent to each other in a planar manner, e.g., to optimize transfer of ions and active agents towards a plurality of teeth or and/or the gum adjacent to a plurality of teeth. The battery advantageously optimizes the dose delivery of at least one active agent towards at least one tooth, the gum, and/or the lips or tongue of the subject.

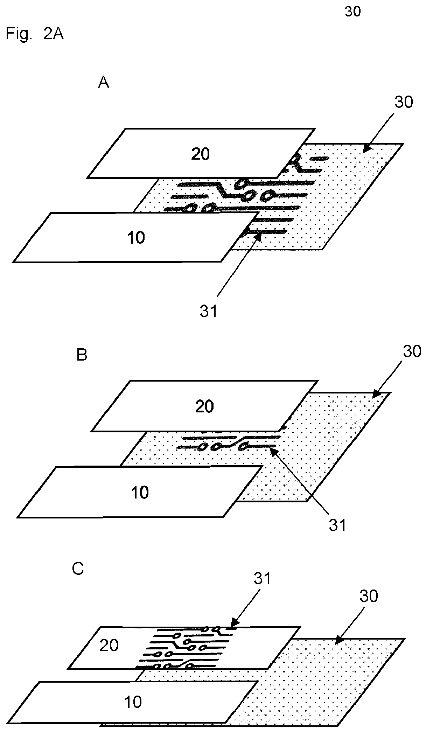

The battery can be embedded in the middle layer or, preferably, the backing layer. The bio-battery may comprise at least one, optimally, two flat meshed electrode embedded within the layer. If the battery is a biobattery, an embedded enzymatic bio-battery is preferred. The layer holding the battery is typically not degradable, but the layer comprising the first and second regions may be degradable, thus allowing for enhanced delivery of active agents via the first surface to the gums and/or teeth. As shown in Fig. 2, the battery may act on the first and the second region, or on the first or the second region. It can be embedded in the backing layer and act on the first and the second region, or on the first or the second region. Alternatively, it can be placed on the back side of the sheet or sheets holding the first and/or second region, as a middle layer.

Alternatively, instead of a battery and at any of the places described for the battery, the film of the invention can comprise an electrode that can be linked to an external battery and that can be used in combination with a further external electrode to provide controlled ion transport to the tooth surface and/or the gum.

In the context of the invention, different sheets or layers can be attached to each other by physical (e.g., networks interpolation, or hydrophobic interaction, ionic interactions) and/or chemical interactions (e.g., covalent bonding). They can be e.g., pressed, welded, heat welded, laser welded, ultrasonic welded, 3D-printed, bonded, coated, cast, or chemically linked, e.g., cross-linked to each other.

The oral film or at least one sheet thereof preferably comprises a polymeric material and/or a hydrogel as a matrix material. Matrix material are preferably water-dissolvable, and consequently dissolve when they are in contact with saliva. They are also non-toxic and preferably do not interact with the active agents to an extent that prevents their effect on the teeth or gum.

The matrix material may comprise a vinyl polymer, polyacrylate, PEEK (polyether ether ketone) polymer, carbopol, natural polymer, or copolymers thereof, or pectin coated liposomes. For example, the polymeric material may comprise a natural polymer such as a cellulose-based polymer, e.g., hydroxypropylmethylcellulose (HPMC), hydroxypropylcellulose (HPC), ethyl cellulose (EC), carboxymethylcellulose (CMC) or chitosan, collagen, gelatin, silk, alginate, xanthan gum, albizia gum, or khaya gum. The polymer may be stabilized by chemical and/or physical cross-linking. It may also be an aerogel of self-assembling peptide, e.g., P11-4 or P11-8, or a combination of at least one of the abovementioned polymers with a self-assembling peptide. Additional components to enhance matrix properties (e.g. Dopa (3,4-dihydroxyphenylalanine) may be added.

Natural polymers in a combination with vinyl polymers have been shown to have advantageous characteristics as a matrix material.

Hydrocolloids can encapsulate, protect and release active agents in a controlled manner, e.g, for wounds in the gum or the buccal area. Biocellulose, i.e., cellulose-based polymers may help periodontal regeneration, and has been successfully used for dressing of surgical wounds of the oral mucosa.

The material of the film preferably is at least partially flexible so that the film or at least the first and second regions can be disposed to periodontal or dental therapy regions, respectively. The film may e.g. comprise at least one region that is flexible, wherein the flexible region may be the spacer region. The film may also comprise rigid regions that are less flexible or not flexible, e.g. one or more of the first, second or third regions. Flexibility of the film is preferred, as it allows for a good fit to the teeth and gum, and accordingly, a good transfer of active agent. Alternatively, the film of the invention may have a rigid spatial shape so that the at least one first and at least one second regions form-fit with the periodontal or dental therapy regions of the subject, respectively. Typically, in this case, it is individualized.

The film or the sheet(s) comprising at least the first and second regions may comprise further ingredients in addition to the matrix material and the active agent(s), e.g., plastizier, sweetener, flavour, surfactant, salvia stimulating agent, permeation enhancer, buffer and/or mucosal or dental adhesion enhancer.

In one embodiment, the sheet comprising the first and/or the second (and optionally, the third region) may comprise
- 1-30 wt% of natural polymer, e.g., 3-10 wt% or 5-12 wt%;
- 0-30 wt% of copolymer (e.g., vinyl polymer), e.g., 1-10 wt% or 5-10 wt%;
- 0-10 wt% plastiziser, e.g., 1-5 wt%;
- 0.1-50 wt% active agent, e.g., 1-40, 2-30, 3-25, 4-20, 4-15 or 5-10 wt%;
- 0-5 wt% sweetener, e.g., 1-2.5 wt%;
- 0-10 wt% flavour, e.g., 1-5 wt%;
- 0-5 wt% surfactant, e.g., 1-3.5 wt%;
- 0-5 wt% saliva stimulating agent, e.g., 1-2.5 wt% and, optionally;
- water ad 100 wt%.

The film or at least the first surface thereof is at least partially adhesive, i.e. at least an area of said surface, preferably, the whole first surface is adhesive such at it is capable of holding the film on at least one tooth and at the adjacent gum during the residence time of the film. The film can also be designed in such a way to adhere to crowns, bridges and veneers if necessary. Adhesion can be through physical interactions, but also through mixed bonding adhesion. The sheet(s) of the first and second regions, and optionally, the spacer region may optionally comprise an adhesive of synthetic or natural origin, such as polyacrylic acid and/or polyvinylpyrrolidone complexes. Different topographies of the surface may also contribute to adhesion, e.g., topographies as disclosed by Bovone et al., 2021. ACS Biomater Sci Eng. 2021;7(9):4048-4076. doi:10.1021/acsbiomaterials.0c01677; Yang et al., 2020. Adv. Funct. Mater. 2020, 30, 1901693. https://doi.org/10.1002/adfm.201901693; or Pelaez-Vargas et al., 2010, Microsc Microanal. 2010;16(6):670-676. doi:10.1017/S1431927610094158.

In the context of the invention, active agents are not intended to be limited to active agents in the sense of an active agent recognized as active ingredient in the respective market or in the marketing authorization, which will vary depending on the type of product and the applicable legislation, but any compound having a cosmetic, therapeutic, prophylactic or preventive effect on the tooth and/or gum.

In the context of the invention, the first active agent may be selected from the group consisting of a peroxide, an agent capable of generating a peroxide, a self-assembling peptide, CPP-ACP (caseinphosphopeptide - amorphous calcium phosphate), mineral particles, e.g., hy-dropxyapatite, amorphous calcium phosphate, and fluoride. Combinations thereof can also be used, and are indeed preferred, e.g., use of at least two or at least three active agents. If not explicitly stated otherwise, reference to an active agent means reference to at least one active agent. Said first active agent is comprised in the first region of the film, which is intended to be applied to teeth of the subject. Thus, the first active agent is suitable for treatment of teeth. For example, it can be suited for tooth whitening, caries treatment, caries prophylaxis and/or desensitizing sensitive teeth.

Various techniques for tooth whitening are known, but most are based on bleaching with an oxidizing agent such as peroxide. In particular, the majority of tooth whitening products on the market employs hydrogen peroxide (HP). However, in recent years, awareness of side effects associated with bleaching, such as demineralisation, erosion and tooth sensitivity caused by peroxides, has increased (e.g., Dahl et al., 2003, Crit Rev Oral Biol Med 14(4):292-304). Peroxides are also irritant to the gum and can cause inflammations. Other peroxides, such as carbamide peroxide (CP), or agents capable of generating a peroxide, are considered to have a milder effect. It is for example possible to use a combination of HP and CP, or to use phthalimidoperoxycaproic acid (PAP), alone or in combination with HP. If a peroxide or an agent capable of providing a peroxide are used in the oral film of the invention, PAP is preferred.

Self-assembling peptides (SAP) are known for their excellent characteristics in treatment and prevention of caries lesions (e.g., WO 2004/007532 A1). WO 2004/007532 A1 discloses peptides that are capable of forming three-dimensional scaffolds, thereby promoting nucleation of de-novo calcium phosphate. These artificial peptides assemble in one dimension to form beta-sheet, and higher order assemblies such as tape-like assemblies. Three-dimensional supramolecular structures of SAP can be formed, which have an affinity for/ to calcium phosphate.

In the context of the present invention, self-assembling peptides taught in WO 2004/007532 A1, US10/521,628, US12/729,046, US13/551,878, US 14/062,768, or WO2014/027012 A1, which are all fully incorporated herein by reference, are preferred. In particular, self-assembling peptides having a net charge of +2 or -2 at pH 7.5, may be used in monomeric or assembled form.

Self-assembling peptides used in the present invention preferably comprise a sequence of the formula X1-X2-X1-X2-X1, wherein X1 is an amino acid with an acidic side chain and X2 is an amino acid with a hydrophobic side chain. For example, the self-assembling peptide used in the present invention comprises the consensus sequence SEQ ID NO: 1, X1-X2-X1-X2-X1, wherein X1 is independently selected from the group consisting of glutamic acid, aspartic acid, glutamine and ornithine, and X2 is independently selected from the group consisting of alanine, valine, isoleucine, leucine, methionine, phenylalanine, tyrosine, tryptophan and glutamine, or an amino acid sequence having at least 80% identity thereto. Independently selected means that, e.g., X1 in positions 1, 3 or 5 of the sequence above can be different from each other. Of course, they can also be identical.

Preferably, self-assembling peptides used in the invention comprise SEQ ID NO: 2, X1-X2-X1-X2-X1, wherein X1 is independently selected from the group consisting of glutamic acid and ornithine, and X2 is independently selected from the group consisting of tryptophan and phenylalanine.

In a further embodiment, self-assembling peptides used in the invention may comprise SEQ ID NO: 3, X3-F-X1-W-X1-F-X1, wherein X1 is independently selected from the group consisting of glutamic acid and ornithine, and X3 is selected from the group consisting of arginine, glutamic acid and ornithine, wherein X3 preferably is arginine.

Furthermore, self-assembling peptides used in the invention may comprise SEQ ID NO: 4 or, preferably, consist thereof: X4-X4-X3-F-X1-W-X1-F-X1-X4-X4, wherein X1 is independently selected from the group consisting of glutamic acid and ornithine, and wherein X3 is selected from the group consisting of arginine, glutamic acid and ornithine, and wherein X4 is independently selected from the group consisting of glutamine, glutamic acid, serine, threonine and ornithine. X3 preferably is arginine. Independently, X4 preferably is glutamine.

Self-assembling peptides of the invention may comprise SEQ ID NO: 5, or, preferably, consist thereof: Q-Q-R-F-X1-W-X1-F-X1-Q-Q, wherein X1 is independently selected from the group consisting of glutamic acid and ornithine.

Preferably, the self-assembling peptides used in the present invention comprise or consist of a sequence selected from the consensus sequences listed in Table 1 below.

Most preferably, said peptides comprise the specific peptides listed in Table 2 or consist thereof. Of course, self-assembling peptides assembling in combination with another self-assembling peptide, e.g., as disclosed herein, may be formulated in one kit or in one composition.

Peptides of SEQ ID NO: 6, 9, 11, 12, 16 or 17 are particularly advantageous, e.g., as they can be used in relatively low concentrations, they are highly compatible with cells and have beneficial charge distribution.

Preferably, the self-assembling peptide comprises the sequence of SEQ ID NO: 6 or consists thereof. A peptide consisting of a sequence of SEQ ID NO: 6 is also designated P11-4, and is preferred throughout the invention. In another preferred embodiment, the self-assembling peptide comprises the sequence of SEQ ID NO: 9 or consists thereof (P11-8).

The kit or composition of the invention may also comprise at least one self-assembling peptide having at least 45% sequence identity to a peptide consisting of SEQ ID NO: 6. Preferably, the peptide has at least 54%, at least 63%, at least 72%, at least 81% or at least 90% sequence identity to a peptide consisting of SEQ ID NO: 6, or is said peptide. Peptides of the invention are, for example, 11 amino acids in length.

Self-assembling peptides may be modified peptides comprising an Ac-N-terminus and/or NH₂-C-Terminus, preferably, both, or non-modified peptides. As non-blocked forms tend to start a deaminization reaction, the termini of all self-assembling peptides of SEQ ID NO: 1 are preferably blocked to increase stability. In particular, peptides of SEQ ID NO: 6, 9, 11, 12, 16 and 17 may comprise an Ac-N-terminus and NH₂-C-Terminus. SEQ ID NO: 18-29 correspond to modified peptides of the invention.

**Table 1: Consensus sequences of preferred self-assembling peptides**

| **SEQ ID NO** | **Peptide name** | **Sequence** | **Exemplary SAP** |
|---|---|---|---|
| SEQ ID NO: 1 | Consensus sequence 1 | X1-X2-X1-X2-X1, wherein X1 is independently selected from the group consisting of glutamic acid, aspartic acid, glutamine and ornithine, and X2 is independently selected from the group consisting of alanine, valine, isoleucine, leucine, methionine, phenylalanine, tyrosine, tryptophan and glutamine | P11-2, P11-4, P11-5, P11-8, P11-12, P11-13, P11-14, P11-17, P11-19, P11-20, P11-28, P11-29 |
| SEQ ID NO: 2 | Consensus sequence 2 | X1-X2-X1-X2-X1, wherein X1 is independently selected from the group consisting of glutamic acid and ornithine, and X2 is independently selected from the group consisting of tryptophan and phenylalanine | P11-4, P11-8, P11-12, P11-13, P11-14, P11-17, P11-28, P11-29 |
| SEQ ID NO: 3 | Consensus sequence 3 | X3-F-X1-W-X1-F-X1, wherein X1 is independently selected from the group consisting of glutamic acid and ornithine, and X3 is selected from the group consisting of arginine, glutamic acid and ornithine, wherein X3 preferably is arqinine | P11-4, P11-8, P11-12, P11-13, P11-14, P11-17, P11-28, P11-29 |
| SEQ ID NO: 4 | Consensus sequence 4 | X4-X4-X3-F-X1-W-X1-F-X1-X4-X4, wherein X1 is independently selected from the group consisting of glutamic acid and ornithine, and wherein X3 is selected from the group consisting of arginine, glutamic acid and ornithine, and wherein X4 is independently selected from the group consisting of glutamine, glutamic acid, serine, threonine and ornithine. X3 preferably is arginine. Independently, X4 preferably is qlutamine. | P11-4, P11-8, P11-12, P11-13, P11-14, P11-17, P11-28, P11-29 |
| SEQ ID NO: 5 | Consensus sequence 5 | Q-Q-R-F-X1-W-X1-F-X1-Q-Q, wherein X1 is independently selected from the group consisting of glutamic acid and ornithine. | P11-4, P11-8 |

**Table 2: Preferred self-assembling peptides. Positions X1 are underlined**

| **SEQ ID NO** | **Peptide name** | **Sequence (One letter code)** | **% amino acid identity to P11-4** (ClustalW (2.1, standard parameters)) |
|---|---|---|---|
| SEQ ID NO: 6 | P11-4 | QQRFEWEFEQQ | 100 |
| SEQ ID NO: 7 | P11-2 | QQRFQWQFEQQ | 81.8 |
| SEQ ID NO: 8 | P11-5 | QQRFOWOFQQQ | 72.7 |
| SEQ ID NO: 9 | P11-8 | QQRFOWOFEQQ | 81.8 |
| SEQ ID NO: 10 | P11-12 | SSRFOWOFESS | 45.4 |
| SEQ ID NO: 11 | P11-13 | EQEFEWEFEQE | 72.7 |
| SEQ ID NO: 12 | P11-14 | QQOFOWOFOQQ | 63.6 |
| SEQ ID NO: 13 | P11-17 | TTRFEWEFETT | 63.6 |
| SEQ ID NO: 14 | P11-19 | QQRQOQOQEQQ | 54.5 |
| SEQ ID NO: 15 | P11-20 | QQRQEQEQEQQ | 72.7 |
| SEQ ID NO: 16 | P11-28 | OQOFOWOFOQO | 45.4 |
| SEQ ID NO: 17 | P11-29 | QQEFEWEFEQQ | 90.9 |
| SEQ ID NO: 30 | P11-16 | NNRFOWOFENN | 45.4 |
| SEQ ID NO: 31 | P11-18 | TTRFOWOFETT | 45.4 |
| SEQ ID NO: 32 | P11-26 | QQOQOQOQOQQ | 36.4 |
| SEQ ID NO: 33 | P11-31 | SSOFOWOFOSS | 27.3 |

The self-assembling peptides preferably do not have restriction sites for the subject's endo-peptidases. They also do not need to comprise a special recognition motif for cells.

Mineral particles preferably are calcium phosphate particles, e.g., hydroxyapatite particles. As described in WO 2015/044268 A1, a composition comprising 0.4-40 wt% mineral particles, preferably, hydroxyapatite particles having a size of 0.01-50 µm and 0.001-5 wt% of a self-assembling peptide such as these described herein, preferably, P11-4, also have an excellent tooth whitening effect.

Other suitable mineral particles are amorphous calcium phosphate (ACP). ACP, e.g., in the context of CPP-ACP, has also been shown to contribute to tooth remineralisation. WO 2022/084288 A1 shows that a combination of SAP and ACP may accelerate the remineralis-ing action of SAP. Agents capable of producing ACP can also be used as active agents.

It has now been recognized that peroxides or agents capable of generating peroxides, such as PAP and SAP, optionally, in combination with mineral particles as disclosed in WO 2015/044268 A1 may advantageously be combined. The present application thus provides an oral care product comprising peroxides or agents capable of generating peroxides, such as PAP, and SAP, optionally, in combination with such mineral particles. This oral care product, e.g., an oral film of the present invention comprising these agents in the first region, may not only be used for an improved tooth whitening effect, but it also avoids the demineralisation, erosion and tooth sensitivity typically caused by peroxides. It may also contribute to remineralisation of teeth.

Fluorides also improves tooth remineralisation, as inclusion of fluoride in the enamel makes it harder. Fluorides may be, e.g., sodium fluoride or sodium monofluorphosphate with a fluoride content of up to 1500 ppm. Fluorides can be advantageously combined with SAP and combinations of active agents comprising SAP, e.g., to improve caries prophylaxis, i.e., prevention or reduction of the incidence of caries.

The second region, which is capable of contacting a gum of the subject, comprises at least one active agent that is different from the first active agent. However, it may additionally comprise at least one active agent that is also present in the first regions, i.e., a first active agent. The second active agent may be selected from the group consisting of an anti-inflammatory agent, a senolytic agent, a botanical agent, an anti-bacterial agent, and a self-assembling peptide.

In tooth whitening, e.g., involving peroxides, one common problem is irritation and inflammation of the gum adjacent to the treated teeth by the peroxides. This can also contribute to increased tooth sensitivity. It further contributes to aging of the gums. Therefore, it is advantageous to counteract any such risks simultaneously to the treatment of the teeth.

Anti-inflammatory agents can be, e.g., botanical agents or metallic, e.g., gold particles, or non-steroid anti-inflammatory drugs (NSAIDs). Typical senolytic agents are also botanical agents, often, coloured substances that, if applied to the teeth, may lead to tooth staining. However, using the oral film of the invention, it is also possible to employ such potentially tooth staining agents for targeted treatment of the gum.

Anti-bacterial agents can also be botanical agents. They can also be antibiotics.

Table 2 provides exemplary suitable botanical agents and their main treatment effects. Of note, both isolated active agents and plant extracts can be used as active agents, e.g., a water extract, ethanolic extract, methanolic extract, an oil, e.g., sunflower oil extract, propylene glycolic extract, a PEG extract, a hexane extract, a CO₂ extract or a glycerinic extract or dry extract. In the context of the dental care product of the invention, it may be desired to provide a product that does not comprise ethanol, methanol, hexane, PEG or propylene glycol. Thus, a glycerinic extract or dry extract may be advantageously used, e.g., a glycerinic extract. The glycerinic extract may further comprise water. Of course, combinations of botanical agents can also be used.

**Table 2:**

| **Active agent** | **Active ingredients** | **Function** | **Indicated to treat** |
|---|---|---|---|
| Aloe vera | Lignings, saponins (antiseptic quality), vitamins A, C, E, minerals (early wound healind), amino acids (repair and regeneration traumatized tissue), anthroquinones (healing, antibacterial), sugars (immune modulating and anti/inflammatory actions) | Wound healing, tissue repair, anti-inflammatory | Chronic periodontitis |
| Neem extract | Azadiractin (act. Agent), glycosides (antimicrobial), luminols (anti-inflammatory) | Anti-microbial, anti-inflammatory | periodontal disorders /Oral infections and plaque growth inhibition- |
| Tumeric | | Anti-inflammatory, antimicro-bial, antiseptic, wound healing, etc. | Periodontal pockets |
| Propolis | Flavonoid - galancin and hydroxzcinnamic acids like caffeine | Anti-microbial , | Protect against caries and periodontal diseases |
| Pomegranate | | Inhibit microbial adherence in oral cavity | |
| Inulin | Fructooligosaccharides | Prebiotic | Balancing the oral microbiome, fighting against dental plaque Preventing tooth decay Reducing bad breath |
| Tea tree | | antiseptic, anti-bacteria, anti-fungal, antiviral, and anti-inflammatory agent | Helps reducing oral yeast infections Effective against the common oral pathogens: S. aureus, Enterococcus faecalis, E. coli, and C. albicans Does not contribute to increased bacterial resistance |
| Totarol | | antibacteria, antimicrobial, antioxidant and anti-inflammatory; extracted from totara wood | Potent against Strepto-coccus mutants, the major cariogenic organisms present in dental plaque Active against gingivitis and periodontal disease |
| VACCINIUM MACROCARPON FRUIT EXTRACT | | Prebiotic | Promotion of beneficial bacterial biofilms and in-hibition of undesirable bacterial biofilms |

| | | | |
|---|---|---|---|
| References for Aloe vera: (Indian Journal of Oral Sciences (Vol. 3 (2) 2012.; Geetha Bhat et al. J Indian Soc Periodontol. 2011 15(3): 205-209. Reference for Neem extract: IOSR Journal of Pharmacy Vol 3 (4) , 15/21 2013 Reference for Tumeric: Gen Dent 2012 60 (5): e283/7 K Indian Soc Periodontol 2011, 15(1): 35=38 Reference for Propolis: Journal of Pharmaceutical Sciences Vol 96 (8), 2074/2089, 2007 | | | |

Alternative active botanical agents are extracts from a plant of the genus Rhododendron, preferably, of the subgenus *Rhododendron L,* as classified by Chamberlain 1996, advantageously, the extract is from *Rhododendron ferrugineum* (sometimes called alpine rose or alpenrose, snow-rose, or rusty-leaved alpenrose) or *Rhododendron hirsutum,* as described in US 11,154,470 B1.

It has been shown that such an extract has advantageous characteristics, it particular, it is capable of
- inhibiting UVA-induced photo-aging (protein carbonylation)
- exhibiting a senolytic effect, i.e., it eliminates senescent cells while not affecting healthy cells in a negative way. This rejuvenates the entire tissue as senescent cells contribute to inflammation and aging.
- significantly reducing redness and increase elasticity.

The extract further has potent anti-microbial effects, e.g., against bacteria involved in periodontal disease.

The dental care product may comprise, e.g., 0.1-5 wt%, e.g., 0.5-3 wt% of said extract. Use at 1-2 wt% has been found to be advantageous. The extract may e.g., be a leaf extract extracted at a drug to extract ratio of 1:10 to 1:100, preferably, about 1:50, e.g., with glycerine.

The inventors have found that it is particularly advantageous to combine the dental care product of the invention comprising an extract from a plant of the genus *Rhododendron* with an extract of a plant of the genus *Leontopodium.* Probably due to its high content of anti-oxidants, said extract has anti-inflammatory properties and advantageously synergizes with the *Rhododendron* extract in treating the gums of a subject as further explained below, e.g., in treatment of gingivitis, periodontitis and peri-implantitis, as well as in rejuvenating the gums.

Thus, the dental care product further comprises 0.1-5 wt% of an extract of a plant of the genus *Leontopodium,* preferably, an alpine *Leontododium,* such as *Leontopodium nivale,* which is also designated Edelweiß, as described in US 11,154,470 B1.

The dental care product may e.g. comprise 0.5-3 wt% of said extract. Use at 1-2 wt% has been found to be advantageous.

It has anti-oxidant and anti-inflammatory characteristics. Adsorption and elimination of free radicals is improved, which helps to combat aging of the gums. The high content of tannins also has a protective effect. The tannins also improve elasticity and contribute to regeneration of the gums. Flavoinoids protect the vasculature. Phenylpropanoids have adstringent effects. The blood circulation is stimulated, cell activity improved, and thus, in particular, in combination with the active agents from the *Rhododendron* extract, the regeneration of the tissue is stimulated.

Optionally, the dental care product of the invention further comprises an extract of a plant of the genus *Eleutherococcus,* optionally, *Eleutherococcus senticosus,* colloquially called Siberian ginseng, as described in US 11,154,470 B1. It has as anti-inflammatory, anti-irritant and anti-stress activities and improves wound healing. The dental care product of the invention may comprise, e.g., 0.1-5 wt% of said extract of a plant of the genus *Eleutherococcus,* e.g., 0.5-2 wt% of said extract. Typically, 1-2 wt% are comprised, if said extract is present. The extract may e.g., be a root extract extracted at a drug to extract ratio of 1:10 to 1:100, preferably, about 1:50, e.g., with glycerine.

Preferably, the dental care product does not contain ethanol. Thus, in a preferred embodiment, all herb extracts contained are non-alcoholic extracts, e.g., glycerinic, oil extracts or dry extracts. Such glycerinic extracts may also comprise other ingredients or solvents, preferably water, but they do not comprise ethanol. The dental care product of the invention may be halal and/or vegan.

Preferably, in the oral film of the invention, at least the first region comprises a self-assembling peptide as disclosed herein, e.g., P11-4 comprising SEQ ID NO: 1 or a SAP having at least 70% sequence identity thereto, or P11-8. Preferably, the SAP is P11-4. The concentration range of SAP can be, e.g., 5-50 mg/mL. The second region may also comprise the SAP. In this case, the first region and the second region comprise further, different active agents, e.g., the first active agent can be a peroxide or an agent capable of generating a peroxide, e.g., HP/CP or PAP, preferably, PAP. The concentration range of thereof can be, e.g., 2-20 wt%. Optionally, the first region further comprises fluoride and/or mineral, e.g., calcium phosphate particles having the size disclosed herein that makes them suitable for tooth whitening. The second active agent preferably is an anti-inflammatory botanical agent, e.g., as disclosed above. Alternatively or additionally, the second active agent can be a prebiotic to balance the buccal microbiome, e.g., inulin and/or vaccinium macrocarpon fruit extract, e.g., in a concentration range of up to 5 wt%.

The oral film of the invention can also be a pharmaceutical film, i.e., it can be used for therapy and/or prevention (e.g., reduction of the incidence) of at least one disease or disorder. In particular, the oral film of the invention may be for use in therapy or prevention of at least one periodontal or dental disease or disorder selected from the group comprising caries, mucositis, gingivitis, periodontitis, peri-implantitis and tooth discoloration in a subject. Preferably, it is for use in therapy.

The invention also provides a method, preferably, a cosmetic method of treating a subject, using the film comprising contacting at least one tooth of the subject with the first region of the film and contacting at least a region of the gum of the subject with the second region of the film. Upon application, the presence and flow of saliva may activate the fil, i.e., the active agent from the different regions start to get into contact with the respective targeted area (teeth or gums), e.g,. by diffusion. By provision of ions or use of a bio-battery, as disclosed herein, the active agents can also be actively propelled into or onto the target tissue. If the film of the invention, or sheets thereof, are water-soluble, the dissolution of the film also contributes to delivery of the active agents. The effect of the active agents may be sustained for, e.g., 15 min up to an hour or several hours, e.g., depending on the solubility of the film or sheet(s) thereof, until the last of the film is dissolved. A backing layer less permeable or impermeable to the active agent and/or less soluble than the sheet(s) forming the first and second regions may contribute to targeted delivery.

Preferably, only the teeth are contacted with the first region. For example, for treatment of upper teeth, the first region is adhered to the teeth below the line shown in Fig. 4, and the second region (or the spacer, if present) is adhered above the line, wherein the second region predominantly contacts the gum.

The method may further comprise removing the film or the residue thereof that has not dissolved. Of course, if the film or parts thereof are not soluble, it needs to be removed. Removal can be by taking of the film manually, or, e.g., in the case of a partly dissolved film, by washing it off with water or a suitable solvent that may optionally be provided with the film in a kit. For example, if the film is more easily soluble in ethanol than in water, an ethanolic mouthwash can be provided in a kit together with the film to wash away the film or any residue thereof after a desired time of application. The mouthwash may of course comprise active agents, e.g., anti-inflammatory agents and/or agents capable of neutralizing any remaining peroxides.

The invention also provides a method for preparing the film of the invention, comprising:
providing a film comprising a first surface comprising at least one first region and at least one second region and absorbing at least a first active agent in the first region and at least a second active agent in the second region. The backing layer is typically already attached to the sheet or sheets comprising the first and second regions when the active agents are added.

Preferably, however, the method for preparing the film of the invention comprises preparing a first sheet by mixing matrix material of said first sheet with at least a first active agent and preparing a second sheet by mixing matrix material of said second sheet with at least a second active agent.

Optionally, the method comprises attaching one sheet of the first and second sheets partially or totally onto the other sheet to form the first surface, wherein the sheets are overlapping by 3-100%, optionally, by 10-50%. Alternatively, the method comprises attaching a spacer region between the first and second regions, wherein the spacer region is abutting with these regions. The method may also comprise further attaching a spacer region on the first and second regions, wherein the spacer region is overlapping the regions by 10-50%, respectively.

The film is assembled from the first and the second sheet, and if present the spacer, and optionally, the backing layer. If a middle layer is present, e.g., in the case of a biobattery, the film is assembled with the middle layer between the first and/or second sheet and the backing layer.

A third region may, if present, be attached onto the second surface of the backing layer opposite to the first surface.

Alternatively, the method for preparing the film of the invention involves 3-dimensional printing of the film in an individualized manner, taking the size and form of teeth and, optionally, the conditions or disorders and their severity of the subject into account. This allows for most precise targeting of the active agents, as the form of the gingival border can be mirrored by the border between the first and second regions.

The method of preparing the film of the invention may optionally further comprise packaging the film.

### Figure legends

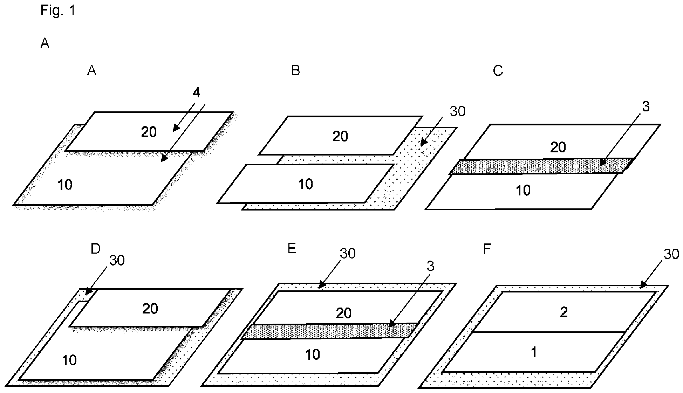

- Fig. 1: shows different embodiments of the film of invention. The different components are slightly removed sideways, which is only for the purposes of better view in the figures. In A, a first sheet 10 comprising the first region 1 overlaps a second sheet 20 comprising a second region 2 by 100%. In B, a first sheet 10 comprising a first region 1 and a second sheet 20 comprising a second region 2 abut each other and are positioned on a backing layer 30. In C, a first sheet 10 comprising a first region 1 and a second sheet 20 comprising a second region 2 abut each other, but at the border between these sheets, a spacer region 3 overlays a part of the first 1 and/or the second 2 region. D shows the same configuration as A, but with a backing layer 30. E shows the same configuration as C, but with a backing layer 30. In A, the first surface 4 is shown with arrows. F shows an embodiment wherein the first region 1 and the second region 2 are on one continuous sheet 10/20, and there is a backing layer 30.
- Fig. 2: shows a dual region multilayer film 5 with a biobattery 31 embedded in the backing layer 30 behind the first and second regions 1, 2 on sheets 10 and 20 in Fig. 2A, behind the second sheet 20 in Fig. 2B, and with separate biobatteries in a middle layer 40 that is to be placed on the backside of the sheet 20 in Fig. 2C. Behind or "the back side" refers to the side opposite the first surface 4. The sheet 20 is shown as transparent, which is not required.
- Fig. 3: shows a dual region multilayer film 5 with a backing layer 30 with ion-impermeability or low ion-permeability. Active agents shown in region 2/sheet 20 are shown to be provided to the gum by ion forced diffusion.
- Fig. 4: Preferably, only the teeth are contacted with the first region 1 of the film 5 of the invention. For example, for treatment of upper teeth, the first region 1 is adhered to the teeth below the line shown, and the second region 2 (or the spacer 3, if present) is adhered above the line, wherein the second region 2 predominantly contacts the gum.

- **1**: first region
- **2**: second region
- **3**: spacer region
- **4**: first surface
- **5**: film
- **6**: second surface
- **10**: first sheet
- **20**: second sheet
- **30**: backing layer
- **31**: battery, e.g., bio-battery
- **40**: middle layer

## Claims

1. A film **5** comprising a first surface **4** comprising at least one first region **1** comprising a first active agent and at least one second region **2** comprising a second active agent different from the first active agent,
wherein the at least one first region **1** is capable of contacting at least one tooth of a subject and the at least one second region **2** is capable of simultaneously contacting a gum of the subject.

2. The film **5** of claim 1, wherein the film **5** is dissolvable after administration of said film **5** in the oral cavity, wherein, preferably, the film **5** is substantially dissolved after 1-240 min.

3. The film **5** of any of the preceding claims, wherein the first region **1** is adjacent to the second region **2** in the first surface **4.**

4. The film **5** of any of the preceding claims, wherein said first region **1** is formed by a first sheet **10** and said second region **2** is formed by a second sheet **20,** wherein surfaces of said first and second sheets (**10**, **20**) together form the first surface **4.**

5. The film **5** of any of claim 4, wherein said first sheet **10** or said second sheet **20** is overlapping 10-100% of the respective other region.

6. The film **5** of claim 4, wherein the first sheet **10** and the second sheet **20** are abutting each other.

7. The film **5** of any of claims 1-2 or 4-6, wherein the first and the second sheets (**10**, **20**) are distanced by a spacer region **3,** wherein the spacer region **3** is configured to inhibit diffusion of at least one of the active agents from one region into the other region,
optionally, wherein the first region **1,** the second region **2,** and, if present, the spacer region **3** are regions of the same sheet forming the film **5.**

8. The film **5** of any of the preceding claims, wherein the first sheet **10** and the second sheet **20** forming the first surface **4** are arranged on a backing layer **30** forming a second surface **6** opposite to the first surface **4,**
wherein, optionally, the backing layer **30** is configured to inhibit diffusion of at least one of the active agents towards the oral mucosa of the subject.

9. The film **5** of claim 8, wherein at least one selected from the group consisting of the backing layer **30,** the spacer region **3,** the first region **1,** and the second region **2** is configured to release ions upon administration to the at least one tooth and the gum, optionally, wherein the film comprises a battery **31,** e.g., a bio-battery.

10. The film **5** of any of the preceding claims, wherein the film **5** or at least one sheet thereof comprises a polymeric material and/or a hydrogel as a matrix material,
wherein the matrix material optionally comprises a vinyl polymer, polyacrylate, PEEK (polyether ether ketone) polymer, carbopol, natural polymer, or copolymers thereof, or pectin coated liposomes.

11. The film **5** of any of the preceding claims, wherein the first active agent is selected from the group consisting of a peroxide, an agent capable of generating a peroxide, a self-assembling peptide, CPP-ACP, mineral particles, and fluoride.

12. The film **5** of any of the preceding claims, wherein the second active agent is selected from the group consisting of an anti-inflammatory agent, a senolytic agent, a botanical agent, an anti-bacterial agent, and a self-assembling peptide.

13. The film **5** of any of the preceding claims, wherein at least the first region **1** comprises a self-assembling peptide comprising the consensus sequence SEQ ID NO: 1, X1-X2-X1-X2-X1, wherein X1 is independently selected from the group consisting of glutamic acid, aspartic acid, glutamine and ornithine, and X2 is independently selected from the group consisting of alanine, valine, isoleucine, leucine, methionine, phenylalanine, tyrosine, tryptophan and glutamine,
wherein the self-assembling peptide preferably has at least 80% sequence identity to SEQ ID NO: 6, and most preferably, comprises SEQ ID NO: 6.

14. A pharmaceutical film **5** of any of the preceding claims,
optionally, for use in therapy of at least one periodontal or dental disease or disorder selected from the group comprising caries, mucositis, gingivitis, periodontitis, peri-implantitis and tooth discoloration in a subject.

15. A method, optionally, a cosmetic method of treating a subject, using the film **5** of any of claims 1 to 14 comprising contacting at least one tooth of the subject with the first region **1** of the film and contacting at least a region of the gum of the subject with the second region **2** of the film.

16. A method of preparing the film **5** of any of claims 1-14, optionally, comprising3-dimensional printing of the film **5** in an individualized manner, taking the size and form of teeth and, optionally, the conditions or disorders and their severity of the subject into account.
